# EUROPEAN PATENT SPECIFICATION

(11) **EP 0 483 730 B1**
(45) Date of publication and mention of the grant of the patent: **07.05.2003**
(21) Application number: 91118360.6
(22) Date of filing: 28.10.1991
(51) Int. Cl.: A61F 13/15

(54) **Absorbent article**
Saugfähige Artikel
Article absorbant

(30) Priority: 29.10.1990 BR 9005475
(43) Date of publication of application: 06.05.1992
(73) Proprietor: McNEIL-PPC, INC., Milltown New Jersey 08850 (US)
(72) Inventor: Costa, Rogério, Laurena, Sao Paulo (BR)
(74) Representative: Groening, Hans Wilhelm, Dipl.-Ing.

(56) References cited:
- EP-A- 0 278 601
- EP-A- 0 349 795
- DE-A- 3 830 056
- FR-A- 2 628 761
- US-A- 4 433 972
- US-E- 32 957

## Description

The present invention refers to a catamenial pad in accordance with the preamble of claim 1 of which the features are known from US-E-32 957.

US-E-32 957 discloses an absorbent article comprising a fluid pervious cover layer, an absorbent core and a fluid pervious barrier layer. A number of individual elements have shapes that allow each of said elements to be movable with respect to other such elements while at the same time permitting an existence of voids between the individual elements. Said elements are essentially resistant to collapse of their individual shape and are confined inside a physical envelope being substantially permeable to liquids. The 'envelope is provided by at least one pocket containing a uniform admixture of discrete superabsorbent particles and discrete introfying particles. The pockets being made from permeable material and formed as quilts or honeycomb like cells are sandwiched between the facing and backing layers. Each pocket contains a substantially uniform admixture of hydrocolloid particulate material and introfying particles. The introfying particles cooperate to maintain the hydrocolloid particles in spaced relationship thereby preserving the interstitial network between the hydrocolloid particles and permitting liquid to circulate through the void areas and contact the exposed surfaces of the hydrocolloid particles. The introfying particles prevent adjacent hydrocolloid particles -from coalescing when they are wetted and begin to swell, thereby maximizing utilization of the large absorptive capacity of the hydrocolloid particles.

Catamenial pads articles are items of general consumption in modern life, that are commercially available and widely used by consumers, and that are the subject of intensive research and development by many companies.

Catamenial pads, as sanitary napkins, in their simplest and most usual form comprise an absorbent core placed between a fluid permeable facing sheet and a fluid impermeable backing sheet.

The fluid permeable facing sheet (also called top sheet or cover sheet) is intended for surface contact with the user's body, allowing the fluid expelled from the body to pass rapidly therethrough towards the absorbent core. This facing sheet is usually a polymer-based nonwoven cloth, with or without perforations, and can be either hydrophilic or, preferably, hydrophobic. In the latter case, the liquid passes therethrough, but the sheet tends to remain dry to the touch and hardly be stained, due to its low or absent hydrophylicity, thus making the product more comfortable and appealing to use. Nonwoven cloth, if not made from hydrophobic fibers, can be rendered hydrophobic by treatment with, e.g., repellent finishes as provided by fluorochemicals, wax emulsions, and the like. Alternative material for facing sheets are perforated plastic films, polymeric foams, etc.

The absorbent core (also called absorbent panel or absorbent layer) is designed rapidly and uniformly to absorb and contain liquids arriving through the facing sheet. The absorbent core may comprise comminuted wood-pulp fibers, polymer foams, vegetable pulps, natural or synthetic fibers, or any other material which can absorb menstrual fluids and other body discharges.

The fluid impermeable backing sheet is intended to prevent the fluid collected by the absorbent core from leaking out of the sanitary pad and staining the clothing of the user. The backing sheet usually comprises a thin film of plastic such as polyethylene, although in many cases it might also comprise other materials, e.g., a nonwoven cloth which has been suitably treated so as to be liquid impermeable. If desired, the backing sheet may be moisture vapor permeable, that is, it may allow moisture vapor to pass therethrough while at the same time preventing the passage of liquids.

A problem concerning sanitary napkins known and/or body odor to those skilled in the art is the occurrence of side leakages during use, which cause discomfort and embarrassement to the user. Furthermore, to the eyes of the consumers, the article is depreciated since it fails to fulfill the absorption function it is intended to perform.

One of the factors that lead to leakage is the low liquid-retention power of the materials used in the absorbent core, very commonly wood-pulp fibers. The liquid already absorbed is expelled with the pressure created by the movements of the user, since the absorbent core is easily compressed and deformed. The greater the amount absorbed, the worse the problem.

Another cause of failure leading to leakage is due to the also well known fact that wood-pulp fiber structures deform or lose their shape under wet conditions. Nevertheless, wood pulp is frequently used to impart shape to some commercial disposable napkins, for instance in the form of a protuberance in the 'central area, in an effort'to close the gap between the body and the absorbent medium. In such a case, what initially had an anatomical shape in the dry state, collapses in contact with wetness and turns into a formless mass even in the absence of pressure. Needless to say, this phenomenon greatly favors leakage.

Still another cause of leakage is the existence of gaps between the catamenial pad, when in use, and the body of the woman. Unlike what is stated in Brazilian Patent PI 8500769 (page 4, lines 24-25), little or no perineal penetration allows menstrual blood or other discharges to spread over the vaginal lips when leaving the vaginal channel, due to the capillarity effect. This creates an increasing possibility of liquid reaching the regions of the sanitary napkin where less or no absorbent material is available, and eventually leaking. There are commercial articles with a thicker central protuberant area made of wood pulp, aiming at reducing the distance between the body and the absorbent medium. As already mentioned, however, such improvement is not efficient because contact with liquids causes the shaped vegetable-pulp fiber block to collapse, and the problem remains.

The catamenial pads of the present invention help to minimise or eliminate leakages that commonly occur with the disposable catamenial pads known in the prior art by:
- dynamically adapting their shape to the otherwise free space between the body of the user and the absorbent medium, thus avoiding the flow of liquid along the skin towards the edges of the absorbent medium;
- and resisting the collapse of its structure either 5 when humid or when under mechanical constraints.

In view of the problems existing in the prior art applicant has created through its research a disposable catamenial pad that is simple and effective, with a low cost/performance ratio, which eliminates or greatly reduces the shortcomings cited above.

Thus one object of the present invention is to provide a disposable catamenial pad, that decreases or eliminates the distance between the body of the user and the absorbent medium of said article, conforming itself to whatever shape is necessary to that end.

Another object of the present invention is to provide a conformable catamenial pad that does not collapse in the presence of wetness or under pressure during use.

A further objective of the invention refers to catamenial pads which should be light, comfortable in use, and effective against leakages.

One of the advantages obtained by the catamenial pads of the invention is that, since the menstrual blood is a visco-elastic fluid, elements contained in the conformable structure help to distribute the flow more uniformly inside the absorbent because of the paths created by the voids among the individual elements. In the. absence of these voids or empty spaces, as is the case of the catamenial pads of the prior art, the blood tends to remain coherent, concentrating about the region where it is discharged, unable to be absorbed as fast as is necessary, especially when the flow is plentiful, and this situation favours leakage.

### SUMMARY OF THE INVENTION

The present invention achieves the above-mentioned objects by the features as disclosed in the characterizing portion of claim 1.

In accordance with the invention, the conformable disposable catamenial pad comprises a number of individual elements having a substantially spherical, rounded or ovaloid shape such that each of the said elements can move with respect to other such elements while at the same time permitting the existence of voids between individual such elements, even when the said elements are subject to mechanical constraint, said elements being essentially resistant to collapse of their individual spacial shape, both due to a mechanical constraint and due to contact with humidity.

Other benefits and advantages brought up by this invention will be disclosed in the following description.

### Brief Description of the Figures

Figure 1 shows a cross sectional view of an absorbent article in accordance with a first embodiment of the invention;
Figure 2 shows a cross sectional view of an absorbent article in accordance with a second embodiment of the invention;
Figure 3 shows a cross sectional view of an absorbent article in accordance with a third embodiment of the invention;
Figure 4 shows a cross sectional view of an absorbent article in accordance with a fourth embodiment of the invention;
Figure 5 shows a cross sectional view of an absorbent article in accordance with a fifth embodiment of the invention;
Figure 6 shows a cross sectional view of an absorbent article in accordance with a sixth embodiment of the invention;
Figure 7 shows an upper view of an embodiment of an envelope for the individual elements that constitute the conformable structure of the invention;
Figure 7B shows a side view of figure 7A;
Figure 8A shows an elevational side view of a catamenial pad as to the shapes it assumes before and after use; and
Figure 8B shows an upper view of catamenial pad as to the shapes it conforms before and after its use.

### Detailed Description of the Figures

The accompanying figures are illustrations of possible configurations of the present invention, their proportions and dimensions being intentionally distorted so as better to describe the invention.

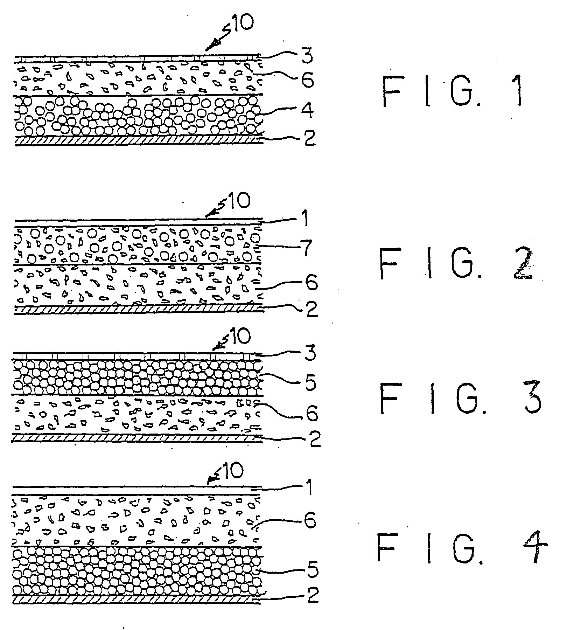

In one embodiment of the present invention the individual elements that constitute the conformable structure are hydrophobic. Only by way of illustration figures 1 and 2 depict catamenial pads containing at least one such conformable structure.

Different configurations for the catamenial pad 10 can be observed in such figures. In figure 1, a perforated plastic sheet 3 is provided which is located at the region of contact with the user. Adjacent to the referred sheet are a wood-pulp pad 6, a plurality of hydrophobic beads 4 and an impermeable backing sheet which will establish contact with the user's clothes. In the configuration which is shown in figure 2, the resulting catamenial pad 10 incorporates characteristics that are included in the previous configuration. Such catamenial pad 10 is made up of a top sheet of nonwoven cloth 1, a plurality of hydrophobic beads dispersed in wood-pulp 7, a wood-pulp pad 6 and an impermeable backing sheet 2.

According to another embodiment of the present invention,the individual elements are hydrophilic. This is exemplified in figures 3 to 6.

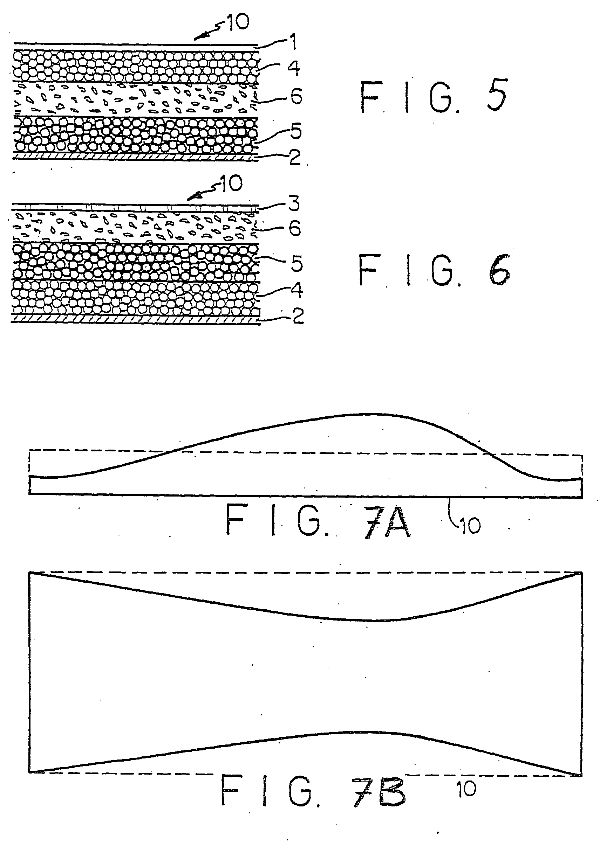

In the configuration of figure 3, a perforated plastic sheet 3, a plurality of hydrophilic beads 5, a wood-pulp pad 6 and an impermeable backing sheet 2 are provided. The configuration of figure 4 is provided with a top sheet of non-woven cloth l, a layer of wood-pulp 6, a plurality of hydrophylic beads 5 and an impermeable backing sheet 2.

In a further variation, the catamenial pads of the invention contain more than one conformable structure, as exemplified in figures 5 and 6. These variations constitute a combination of the configurations which are shown in previously described figures 1 through 4.

The catamenial pads of the present invention do not exclude any other materials and processes known to those skilled in the art, such as:
- the use of superabsorbent materials
- the use of non-fibrous vegetable absorbent materials;
- hydrophylic or hydrophobic top or backing sheets;
- nonwoven or plastic top or backing sheets;
- single or multilayered top or backing sheets;
- embossed, flat or perforated top or backing sheets;
- a single or multilayered absorbent core;
- a multi-density or mixed-material absorbent core, etc.

Figure 7 shows the shape of an envelope 9 suitable for containing individual elements that constitute the conformable structure, which can be positioned close to or on other layers that constitute a disposable catamenial pad. The envelope 9 is symmetrical with respect to axes XX and YY and is composed of two overlapping and peripherically sealed sheets, this seal being represented by the line between points 11 and 12 in figure 7B.

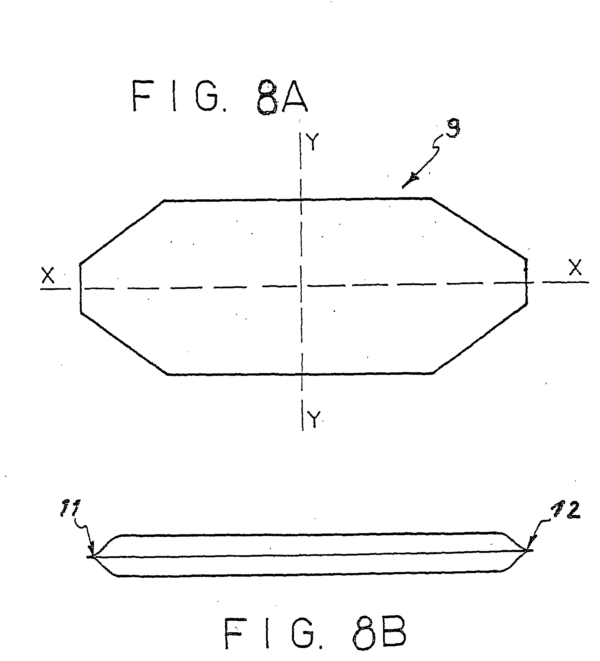

Figures 8A and 8B show the conformation of the catamenial pad before and after its use. The broken line represents the physical aspect of the catamenial pad 10 just before use. The continuous lines represent the physical aspect of the same catamenial pad 10 after being used wherein one can observe the resulting anatomical shape.

### Description of the Invention

In its simplest reduction to practice, the catamenial pad is a plurality of spherical particles enclosed in a liquid permeable envelope.

The catamenial pad of the invention has the ability to conform itself to the space within which it is confined, through the relative movements of its individual elements.

By "individual elements", in the sense employed herein, are meant physically separate elements. But it may also express a relative expression of unity, that is to say, it is possible that a few individual elements agglomerate randomly by force of the conditions prevailing during use, and form "macroelements" whose performance is equivalent to that of individual elements, that is to say: mobility with respect to other elements, resistance to collapse under stress or wetness, and empty spaces between the elements. For example, some individual elements may be kept together by the surface tension of menstrual blood. Nonetheless, the group of elements so formed behaves as an individual element.

By "a number of individual elements" as mentioned herein is meant a minimum amount of said elements that allows at least one of the following facts to occur:
- the conformation of the catamenial pad to the body of the user;
- better resistance to collapse of the catamenial pad that comprises the conformable structure, in comparison with a wood-pulp fiber pad subject to the same conditions.

By "mechanical constraints" as used herein are meant the stress forces such as traction, compression, stretching, shearing, etc., as imposed either by the movements of the user or her clothes upon the catamenial pad.

By "voids" as used herein is meant the room available for fluid to flow among the individual elements that make up the conformable structure, even with maximum packing of the individual elements. Such empty spaces or voids also provide the structure of the invention with aeration channels, since they allow air or moisture vapor to circulate when not totally filled with liquid.

By "essentially resistant to collapse of the individual spacial shape" as used herein is meant the fact that, even under maximal deformation of the individual elements that make up the conformable structure, there is not any total occlusion of the voids among said individual elements. This also means that the elements do not necessarily have rigid structures, as some flexibility or softness is also accepted.

Rounded, ovalled or spherical elements are used with shapes that approach that of, for instance, grains of rice, beans, or sausages.

It is possible to combine elements having different shapes within the same conformable structure. For example, ovalled elements together with spherical ones, or any other desired combination.

A great variation can be applied to the size of the elements of the catamenial pad of the invention. For instance, in the case of spherical elements, the diameters can be either equal or different among the individual elements. When rounded or ovaloid, elements are used their dimensions do not exceed 10 mm, and in case of spheres diameter should be between 0.1 and 5 mm, and most preferably between 0.3 and 1.0 mm.

There is no restriction as regards the combination of different sizes and shapes of the elements that make up the catamenial pad. Preferably only spheres are employed with diameters between 0.3 and 0.5 mm.

The density of the material of the individual elements is not of essential importance; it is up to those skilled in the art being able to select the most adequate value in view of the intended use since performance, comfort and presentation are usually the properties one wants to impart to disposable absorbent articles. Materials with low density, around 0.1 g/cm3 are adequate for catamenial pads particularly around 0.005 g/cm3 and preferably between around 1.0 and 1.0 g/cm3.

The materials and the elements of the conformable structure are made of or can be hydrophobic or hydrophilic, as the case may require. Examples of useful hydrophobic materials used as elements are: synthetic polymers such as polystyrene, polyethylene, or polypropylene, also in the form of aerated or expanded beads; synthetized glass, bakelite, rubber, agglutinated silica, all also under the form of aerated or expanded beads, etc.

As examples of hydrophilic materials useful as elements are: active ceramics, wood or cellulose agglomerates optionally expanded or aerated, vermiculite, vegetable seeds, etc.

Combination of elements made of different materials is possible, as well as use of simultaneous use of elements with different hydrophylicities.

Preferably expanded polystyrene beads are used.

The individual elements of the catamenial pad perform further functions than only imparting shape to a catamenial pad, as they have been previously treated to have properties such as neutralizing or masking body odours, absorbing ions, attacking microrganisms, neutralizing ammonia, coagulating menstrual blood, lubrication, etc.

In the same way, hydrophobic elements may be treated to have surface hydrophylicity or vice-versa, and it is up to 5 the person skilled in the art to conform the material to the use required. Advantageously expanded polystyrene can be treated with a surfactant to become surface-hydrophylic.

Advantageously styrene beads as the individual elements are treated with surfactant and baking soda solution to neutralize menstrual odors.

It is also possible to lubricate the elements to obtain increasing capacity of the conformable structure to quickly adapt its shape to the anatomy of the user under any circumstances, and to decrease eventual ruffling sounds.

As already mentioned, the conformable structure conforms itself to the space it is confined in through the relative movements of the individual elements - and for the most part this property of conformation is transmitted to the catamenial pad that contains the conformable structure. Thus, the user positions the catamenial pad over her vaginal region, causing the pad to occupy the adjacent space, even if the latter modifies itself with the movement of the user. This reduces the possibility of leakage, provides more comfort and arouses the feeling of assurance against leakage.

The volume of the conformable structure has to be adapted to the overall structure of the pad it takes part in. In an advantageous manner, it is adequate to substitute a fraction of the absorbent core total volume by the individual elements of the conformable structure.

The elements of the conformable structure are confined within a physical envelope that will limit its spread without limiting its contact with the liquid to be absorbed.

A suitable material of the envelope is a perforated nonwoven cloth, or preferably a perforated plastic cloth, with openings that are small enough to hold said elements. The shape of this envelope can be any, but symmetrical ones for ease of use are preferred.

In the catamenial pad that comprises facing sheet, absorbent core and backing sheet, the conformable structure in the form of an envelope is preferably placed closer to the body of the user, for instance, right below the facing sheet, where it can best perform its conforming ability. However it is also possible to place it inside the absorbent core, between the core and the backing sheet, etc. In this case, the limitation is dictated only by the use the catamenial pads according to the invention are intended.

The invention is not limited to the conformable structure not being physically connected to the rest of the catamenial pad.

The catamenial pad of the present invention may contain more than one conformable structure. For instance, a first hydrophylic conformable structure superimposed to a hydrophobic one, each performing different tasks as regards the affinity for liquids. Any other combination regarding the number of conformable structures are within the scope of the invention.

5 Illustrative examples are given below with a view further to illustrate the invention, but it is understood they are not intended in any way to limit the scope thereof.

### I - Auto conformation to the body of the user test

An in-vivo test was run with 15 women who received each a set of 10 sanitary napkins from the prior art (articles A) and 10 sanitary napkins according to the invention (articles B). A detailed description of articles A and B will be made later.

Napkins A and B were used alternatively by the women, according to their normal habits, during their menstrual periods.

After use of each napkin, in order not to alter the shape thereof, the user's undergarment was cut at the waist, and the undergarment plus napkin combination was put in a plastic container, hermetically sealed and taken to the laboratory to be analysed.

### Results

- The articles A - napkins according to the prior art - did not show, after use, any defined shape common to all components of this group, while the napkins according to the invention, articles B, were lighter and showed consistently the shape depicted in figures 7A and 7B.
- As to leakage, while articles A of the prior art showed incidence of around 10% failure, only 4% of articles B according to the invention leaked.

Such results lead to the conclusion that the articles according to the invention are auto-conformable to the body of the user, lighter and less prone to leakage than prior art articles.

### Description of Articles A and B

- Articles A - napkins according to the prior art, composed of 3 superimposed layers, in the following order:
   - cover sheet
   - absorbent panel
   - backing sheet

Cover sheet: 100% polyester nonwoven cloth, waterjet perforated, with 144 perforation per square inch, grammage 22 g/m2, containing ethylene-acrylic acid binder to fix the fibers. Its dimensions are enough to wrap the absorbent core/backing sheet group, being heat-sealed onto itself by the backside of the impermeable backing sheet as well as near. the longitudinal extremities of the absorbent core.

Absorbent panel: 100% fluff wood-pulp bleached fibers, with long fibers, weight 9 g, dimensions 185 x 65 x 15 mm, density 0,1 g/cm3.

Backing sheet: impermeable polyethylene film 19 g/cm2.
- Articles B: according to the present invention - article composed of 4 superposed layers in the following order:
   - cover sheet
   - absorbent panel
   - conformable body
   - backing sheet

Cover sheet: as described for articles A.

Absorbent panel: as described for articles A, except for the weight brought down to 3 g, keeping the same dimensions as regards width and length, proportionally decreasing thickness.

Conformable body: 1,5 g polystyrene beads (0.7 g/cm3 specific gravity, 0,025 g/cm3 bulk density, 0,3-0,5 mm diameters) made by BASF in Brazil, under the name Styropor, reference P301. The beads are contained in an envelope of 20% open area perforated polyethylene film, 35 g/m2 grammage, with flat EHD=0,351 mm perforations (EHD = Equivalent Hydraulic Diameter), heat-sealed along its periphery with the shape depicted in fig. 11 (flat perforations designate perforations with approximately cylindrical walls and caliper approximately equivalent to the thickness of the film).

Impermeable backing sheet - as described for articles A.

Flat perforations designate perforations having approximately parallel walls and also having a height that is equivalent to the thickness of the film. The equivalent Hydraulic Diameter (EHD) is the diameter of a circular opening having characteristics of fluid flow that are similar to an irregular opening for which a calculation is made; the EHD is given by EHD=4A/P where A is the opening area and P is the opening perimeter.

### II - Performance test

Two absorbent articles C and D were tested regarding three features: penetration time, rewetting and stain area. Such articles are described hereafter.

Absorbent article C: known in the prior art, composed of 5 superposed layers in the following order:
1. Cover sheet - 20% open area polyethylene perforated film, 35 g/m2, with flat EHD=0,351 mm perforations.
2. Wood-pulp fibers: as described in the previous example, 70 cm3 volume.
3. Nonwoven cloth: non-apertured 200 g/m2 rayon.
4. Superabsorbent laminate: sodium polyacrylate put between two sheets of tissue paper, 70 g/m2 (50% superabsorbent and 50% tissue paper).
5. Impermeable backing sheet: as described in the previous example.

Article D: Identical to article C except that in the layer 2 above the wood-pulp fibers are replaced by 70cm3 polystyrene beads, already described in example 1.

The test procedures for penetration time, rewet and stain area are now described.

### PENETRATION TIME

15 cm3 of Med's fluid are poured at once onto the absorbent structure through an oval artifice (3.9 x 2.6 cm) in an acrylic plate (27 cm x 11 cm x 7.5 mm, 276.4 g weight) placed over the article.

The time the fluid takes to be absorbed, measured in seconds, is called Penetration Time.

### REWETTING

After the Penetration Time test, a uniform 0,5 psi load (aprox. 34 g/cm2) is applied to an area of 120 cm2 of the article, for 3 minutes. The load is suspended, and two previously weighted sheets of filter paper (FRAMA brand, 80 g/cm2, 12,5 cm diameter) are placed on that same area and the load is applied again for 1 minute.

### STAIN AREA

The dimensions the stain left on the filter paper after the Rewet test (indicated by the maximum width and maximum length) are designated Stain Area value.

### RESULTS

Table A below shows the results obtained in the mentioned tests:

**TABLE A**

| Structure | Penetration time(s) | Rewet (g) | Stain area (cm) |
|---|---|---|---|
| C | 9.4 | 1.14 | 9.5 x 6.3 |
| D | 5.3 | 0.37 | 5.0 x 3.3 |

Each value in table A represents the average of three tests.

### ANALYSIS OF THE RESULTS

A comparison of the results show important improvements obtained by the invention (article D) over the prior art article (article C).
- With penetration time one measures how fast the article can acquire external liquid. Article D took less time to absorb liquid than article C, that is, it allowed liquid to penetrate faster.
- The rewet test shows the liquid retention capacity of the article when under pressure. Article D was able to hold liquid more efficiently than article C.
- The stain area test represents the ability of the structure to keep fluids away from its surface. In that sense, on article D a smaller spot remained on the surface of article D than on article C.

## Claims

1. A catamenial pad for feminine hygiene comprising:
- a fluid permeable facing sheet' (1; 3), an absorbent core (6) and a fluid impermeable backing sheet (2) ;
- at least one conformable structure made up of individual elements (4; 5) having a substantially spherical, rounded or ovaloid shape such that each of the said elements (4; 5) can move with respect to other such elements while at the same time permitting the existence of voids between individual such elements, even when the said elements are subject to mechanical constraint;
said elements being essentially resistant to collapse of their individual spacial shape, both due to a mechanical constraint and due to contact with humidity and
- said individual elements (4; 5) are confined inside a physical envelope (9) being substantially permeable to liquids;
- the individual elements (4, 5) having either hydrophobic properties and comprising material selected from the group consisting of synthetic polymer glass bakelite, rubber, silica, ceramics, or having hydrophylic properties and comprising material selected from wood, cellulose agglomerations, vermiculite, and vegetable seeds; **characterized in that**
- the individual elements, when being rounded or ovaloid, have maximum dimensions less than 10 mm and
- the individual elements, when being substantially spherical, have diameters between 0.1 and 5.0 mm,
- the individual elements (4, 5) have been treated to have as further function an activity of neutralization or masking of the body odor and/or ion absorption and/or microorganism attack and/or neutralization of ammonia and/or blood coagulation and/or lubrication.

2. The catamenial pad of claim 1, wherein the individual substantially spherical elements have diameters between 0.3 mm and 0.5 mm.

3. The catamenial pad of claim 1, wherein the individual elements (4, 5) have densities between 0.005 g/cm³ and 1.0 g/cm³.

4. The catamenial pad of claims 1 to 3, wherein the individual elements (4, 5) have densities between 0.1 g/cm³ and 1.0 g/cm³.

5. The catamenial pad of claim 1 to 3, wherein the individual elements (4, 5) comprise material selected from the group consisting of polystyrene, polyethylene, polypropylene, bakelite, rubber.

6. The catamenial pad of claim 1, wherein the envelope (9) is a nonwoven cloth.

7. The catamenial pad of claim 1, wherein the fluid pervious facing sheet (1) is a perforated plastic film (3).

8. The catamenial pad of claim 1, wherein the envelope (9) has a symmetric form.

9. The catamenial pad of claim 1, wherein the individual elements (5) have hydrophilic properties and are positioned between the fluid permeable facing sheet (1) and the fluid impermeable backing sheet (2).

10. The catamenial pad of claim 1, wherein the conformable structure (4; 5) is not physically joined to the remaining components of said pad.

## Patentansprüche

1. Eine Damenbinde für weibliche Hygiene umfassend:
- Eine flüssigkeitsdurchlässige Vorderschicht (1; 3), einen absorbierenden Kern (6) und eine flüssigkeitsundurchlässige Rückschicht (2);
- wenigstens eine sich anpassende Struktur, die aus individuellen Elementen (4; 5) gemacht ist, die im Wesentlichen eine sphärische, gerundete oder ovaloide Form haben, so daß jedes der Elemente (4; 5) sich in Bezug auf andere solche Elemente bewegen kann, die gleichzeitig die Existenz von Lücken zwischen solchen individuellen Elementen erlauben, selbst wenn die Elemente Gegenstand von mechanischen Zwängen sind;
- besagte Elemente im Wesentlichen resistent gegen den Kollaps ihrer individuellen räumlichen Form sind, sowohl aufgrund eines mechanisches Zwanges als auch aufgrund von Kontakt mit Feuchtigkeit und
- besagte individuelle Elemente (4;5) innerhalb einer physikalischen Hülle (9), die im Wesentlichen durchlässig für Flüssigkeiten ist, beschränkt sind;
- die individuellen Elemente (4; 5) entweder hydrophobe Eigenschaften haben und Material umfassen, ausgewählt aus der Gruppe bestehend aus synthetischen Polymeren, Glas, Bakelite, Gummi, Silica, Keramiken, oder hydrophile Eigenschaften haben und Materialien umfassen, ausgewählt aus Holz, Celluloseagglomerationen, Vermikulite und pflanzlichen Samen; **dadurch gekennzeichnet, daß**
- die individuellen Elemente, wenn sie gerundet oder ovaloid sind, eine maximale Dimension von weniger als 10 mm haben und
- die individuellen Elemente, wenn sie im Wesentlichen sphärisch sind, Durchmesser zwischen 0,1 und 5,0 mm haben,
- die individuellen Elemente (4, 5) behandelt worden sind, um als weitere Funktion eine Aktivität zur Neutralisierung oder Markierung von Körpergeruch und/oder Ionenabsorption und/oder Angriff auf Mikroorganismen und/oder Neutralisierung von Ammoniak und/oder Blutgerinnung und/oder Schmierung zu haben.

2. Die Damenbinde nach Anspruch 1, **dadurch gekennzeichnet, daß**
die individuellen, im Wesentlichen sphärischen Elemente einen Durchmesser zwischen 0,3 mm und 0,5 mm haben.

3. Die Damenbinde nach Anspruch 1, **dadurch gekennzeichnet, daß**
die individuellen Elemente (4, 5) eine Dichte zwischen 0,005 g/cm³ und 1,0 g/cm³ haben.

4. Die Damenbinde nach den Ansprüchen 1 bis 3, **dadurch gekennzeichnet, daß**
die individuellen Elemente (4, 5) eine Dichte zwischen 0,1 g/cm³ und 1,0 g/cm³ haben.

5. Die Damenbinde nach den Ansprüchen 1 bis 3, **dadurch gekennzeichnet, daß**
die individuellen Elemente (4, 5) ein Material umfassen, ausgewählt aus der Gruppe bestehend aus Polystyrol, Polyethylen, Polypropylen, Bakelite, Gummi.

6. Die Damenbinde nach Anspruch 1, **dadurch gekennzeichnet, daß**
die Hülle (9) ein nicht gewebter Stoff ist.

7. Die Damenbinde nach Anspruch 1, **dadurch gekennzeichnet, daß**
die flüssigkeitsdurchlässige Vorderschicht (1) ein perforierter Plastikfilm (3) ist.

8. Die Damenbinde nach Anspruch 1, **dadurch gekennzeichnet, daß**
die Hülle (9) eine symmetrische Form hat.

9. Die Damenbinde nach Anspruch 1, **dadurch gekennzeichnet, daß**
die individuellen Elemente (5) hydrophile Eigenschaften haben und zwischen der flüssigkeitsdurchlässigen Vorderschicht (1) und der flüssigkeitsundurchlässigen Rückschicht (2) positioniert ist.

10. Die Damenbinde nach Anspruch 1, **dadurch gekennzeichnet, daß**
die sich anpassende Struktur (4; 5) nicht physisch an die restlichen Komponenten der Binde gebunden ist.

## Revendications

1. Serviette hygiénique pour l'hygiène féminine comprenant : un voile externe perméable aux fluides (1, 3), une partie centrale absorbante (6) et un voile interne imperméable aux fluides (2) ; au moins une structure conformable composée d'éléments individuels (4, 5) ayant une forme en grande partie sphérique, arrondie ou ovaloïde de telle sorte que chacun desdits éléments (4, 5) peut bouger par rapport aux autres dits éléments tout en permettant dans le même temps l'existence de vides entre ces éléments individuels, méme lorsque lesdits éléments sont soumis à une contrainte mécanique ; lesdits éléments étant essentiellement résistants à l'affaissement de leur forme spatiale individuelle, dû à la fois à une contrainte mécanique et au contact avec l'humidité et lesdits éléments individuels (4, 5) sont enfermés à l'intérieur d'une enveloppe physique (9) considérablement perméable aux liquides ; les éléments individuels (4, 5) ayant des propriétés hydrophobes et comprenant un matériau choisi dans le groupe composé de polymère synthétique, de verre, de bakélite, de caoutchouc, de silice, de céramique, ou ayant des propriétés hydrophiles et comprenant un matériau choisi dans le groupe composé de bois, d'agglomérations de cellulose, de vermiculite, et de semences de légumes ; **caractérisée en ce que** les éléments individuels, lorsqu'ils sont arrondis ou ovaloïdes, ont des dimensions maximum inférieures à 10 mm et **en ce que** les éléments individuels, lorsqu'ils sont en grande partie sphériques, ont des diamètres compris entre 0,1 et 5 mm, **en ce que** les éléments individuels (4, 5) ont été traités pour avoir comme autre fonction une action de neutralisation ou de masquage de l'odeur corporelle et/ou d'absorption ionique et/ou d'attaque de microorganismes et/ou de neutralisation de l'ammoniac et/ou de la coagulation sanguine et/ou de la lubrification.

2. Serviette hygiénique selon la revendication 1, dans laquelle les éléments individuels en grande partie sphériques ont des diamètres compris entre 0,3 et 0,5 mm.

3. Serviette hygiénique selon la revendication 1, dans laquelle les éléments individuels (4, 5) ont des masses volumiques comprises entre 0,005 g/cm³ et 1,0 g/cm³.

4. Serviette hygiénique selon l'une quelconque des revendications 1 à 3, dans laquelle les éléments individuels (4, 5) ont des masses volumiques comprises entre 0,1 g/cm³ et 1 g/cm³.

5. Serviette hygiénique selon l'une quelconque des revendications 1 à 3, dans laquelle les éléments individuels (4, 5) comprennent un matériau choisi dans le groupe composé de polystyrène, de polyéthylène, de polypropylène, de bakélite, et de caoutchouc.

6. Serviette hygiénique selon la revendication 1, dans laquelle l'enveloppe (9) est un non-tissé.

7. Serviette hygiénique selon la revendication 1, dans laquelle le voile externe perméable aux fluides (1) est une pellicule plastique perforée (3).

8. Serviette hygiénique selon la revendication 1, dans laquelle l'enveloppe (9) a une forme symétrique.

9. Serviette hygiénique selon la revendication 1, dans laquelle les éléments individuels (5) ont des propriétés hydrophiles et sont positionnés entre le voile externe perméable aux fluides (1) et le voile interne imperméable aux fluides (2).

10. Serviette hygiénique selon la revendication 1, dans laquelle la structure conformable (4, 5) n'est pas physiquement reliée aux autres composants de ladite serviette.
